# EUROPEAN PATENT SPECIFICATION

(11) **EP 0 397 352 B1**
(45) Date of publication and mention of the grant of the patent: **29.06.1994**
(21) Application number: 90304449.3
(22) Date of filing: 25.04.1990
(51) Int. Cl.: A61L 2/18

(54) **Microbial decontamination**
Mikrobielle Entseuchung
Décontamination microbienne

(30) Priority: 09.05.1989 US 349304
(43) Date of publication of application: 14.11.1990
(73) Proprietor: STERIS CORPORATION, Mentor, Ohio 44060 (US)
(72) Inventor: Schneider, Edward T., Mentor, Ohio 44060 (US); Siegel, Norman L., Mentor, Ohio 44060 (US); Kralovic, Raymond C., Austinburg, Ohio 44010 (US)
(74) Representative: Parr, Ronald Edward R.E. Parr & Co.

(56) References cited:
- EP-A- 0 232 170
- EP-A- 0 332 310
- EP-A- 0 395 296

## Description

The present invention pertains to the art of microbial decontamination. It finds particular application in conjunction with sterilizing medical equipment and will be described with particular reference thereto. It will be appreciated, however, that the invention is also applicable to disinfecting systems as well as to microbially decontaminating a wide range of items, including dental instruments, endoscopes, laboratory equipment, manufacturing equipment, and other equipment and items on which it is desirable to eliminate microbial life forms.

"Sterilization" connotes the absence of all life forms, including bacterial endospores which are the living organisms most resistant to conventional sterilants. "Disinfection", by distinction, only connotes the absence of pathogenic life forms. "Microbial decontamination" is generic to both sterilization and disinfection.

Most medical equipment is sterilized at high temperatures. Commonly, the equipment is sterilized in a steam autoclave under a combination of high temperature and pressure. However, endoscopes, rubber and plastics devices or portions of devices, such as lenses, and the like may be destroyed or have their useful lives severely curtailed by such heat and pressure.

The more sensitive medical equipment is often sterilized with ethylene oxide, which is thermally less severe than steam. The equipment items must be exposed to the ethylene oxide for a relatively long time, on the order of three and a half hours . Thereafter, eight to twelve hours are normally required for de-gassing or desorbing the ethylene oxide from plastics and other materials which are capable of absorbing the ethylene oxide. The pressurization and depressurization cycles of ethylene oxide sterilization may damage lens systems and other delicate instruments. Moreover, the ethylene oxide is relatively expensive. It is sufficiently toxic and volatile that extensive precautions are commonly taken to assure operator safety.

Liquid systems are commonly used for sterilization of heat sensitive or other delicate instruments; they are normally rapid, cost effective and do minimal damage to medical devices. Commonly, a technician mixes a sterilant composition and manually immerses the item to be sterilized. The immersion is timed manually by the technician. Technician variation, liquid sterilant shelf life, and the like raise problems with assurance and reproducibility of the disinfection. Rinsing of the items to remove chemical residues also adds a variable that reduces the assurance of disinfection or sterility. Once the sterilant solution is rinsed, the item is susceptible to reinfection by air borne microbes.

In accordance with the present invention, a new and improved apparatus for use in microbial decontamination is provided which overcomes the above-mentioned problems.

Accordingly, in a first apsect the invention provides apparatus for use in the microbial decontamination of an article using an antimicrobial liquid, characterised in that the apparatus comprises:
a basin (10) having
(i) a vent aperture (60) disposed in an upper portion thereof,
(ii)a drain aperture (50) disposed in a lower portion thereof, and
(iii) an inlet (28) for the antimicrobial liquid;

a lid (12) for selectively sealing the top of the basin when in a closed position and providing ready access to the basin in an open position;
a container (B) for receiving said article to be microbially decontaminated, the container being removably disposed in the basin, the container defining a container liquid receiving inlet (32), a container drain aperture (46), and a nozzle plate (44) which defines a tortuous inlet path (40) between the container liquid inlet and a plurality of apertures (90) defined in and distributed around the nozzle plate, the tortuous inlet path being sufficiently tortuous that a migration of contaminating microbes in ambient air after removal from the basin is inhibited;
an antimicrobial liquid supply means (20, 70, 72) for supplying the antimicrobial liquid;
a rinse liquid supply means (20) for supplying a rinse liquid;
a drain (46) operatively connected with the drain aperture (50) of the basin for draining the antimicrobial liquid and the rinse liquid from the basin;
a means (56) for opening and closing the drain selectively to drain liquid from the basin and to retain liquid in the basin; and
a pump (54) for selectively pumping volumes of antimicrobial liquid from the antimicrobial liquid supply means and rinse liquid from the rinse liquid supply means to the inlet (28) for selectively filling the container (B) and displacing ambient air therefrom.

In a second aspect the invention provides a container for use in the apparatus of said first aspect, which comprises:
a lower shell and a cover therefor;
the lower shell having a bottom (34) and a peripheral wall (86), there being disposed in the bottom (34) a liquid-receiving inlet (32) and a drain aperture (46) for the antimicrobial liquid, the drain aperture being disposed in a first region of the bottom (34) and adjacent the peripheral wall (86);
a plate (44) which, together with bottom (34),defines a tortuous inlet path (40) between the liquid-receiving inlet (32) and a plurality of apertures (90) disposed in and distributed over the plate;
the inlet path (40) being sufficiently tortuous that migration of contaiminating microbes from ambient air is inhibited;
the container cover having a top (80) and a downward depending peripheral wall (82) which engages the lower shell to close the container; and
at least one of the shell and cover being configured to define a tortuous vent path (48) adjacent a second region of the lower shell spaced apart from said first region and such that liquid received in the container displaces air from the container along the vent path (48).

In a third aspect the invention provides apparatus for use in the microbial decontamination of an article using an antimicrobial liquid, characterised in that the apparatus comprises:
a container-receiving basin (10), the basin having a drain aperture (50) adjacent a lower portion thereof and a liquid inlet (28);
a lid (12) for selectively sealing the basin in a closed position and for providing access to the basin in an open position;
a liquid antimicrobial supply means for supplying an antimicrobial liquid;
a rinse supply means for supplying a rinse liquid;
a drain operatively connected with the drain aperture (50) for draining antimicrobial and rinse liquids from the basin;
a tubing system (26) interconnecting the antimicrobial supply means, the rinse supply means and the liquid inlet (28);
a valve (56) for selectively opening and closing the drain to drain liquid from the basin and retain liquid in the basin;
a pumping means (54) operatively connected with the tubing system (26) for selectively pumping at least the antimicrobial liquid;
a container apparatus (B) removably disposed in the basin for holding an item to be microbially decontaminated with the antimicrobial liquid, said container comprising a lower shell and a cover therefor;
the lower shell having a bottom surface (34), a shell peripheral wall (86) and a drain aperture (50) adjacent the basin lowermost portion;
the drain aperture (50) being adjacent a junction between the bottom surface (34) and the shell peripheral wall (86) adjacent a first end of the shell;
the lower shell defines a liquid-receiving inlet (32) in fluid communication with the basin liquid inlet (28);
a nozzle plate (44) which defines a tortuous inlet path (40) between the shell liquid-receiving inlet (32) and a plurality of nozzles or other apertures (90) defined in and distributed around the plate;
the inlet path (40) being sufficiently tortuous that a migration of contaminating microbes from ambient air after removal from the basin is inhibited;
the container cover (80) having a top surface and a downward depending cover peripheral wall (82) which engages the lower shell to close the container; and
at least one of the shell and cover being configured to define a tortuous vent path (48) adjacent a second end opposite the shell first end, from at least an uppermost portion of the container to the basin such that received liquids displace substantially all air from the container through the vent path (48).

Our copending European Patent Application 0 322 310 (filed 17 February 1989, published 13 September 1989) discloses sterilizing apparatus in which an endoscope is coiled in circular and oval paths defined in a removable tray. A lid closes over the tray in a sealed relationship during the sterilizing process. The endoscope is thus positioned directly between the tray and the lid. In consequence, once the lid is opened, the endoscope is freely accessible to airborne microbes and subject to recontamination.

On the other hand, in the present application, the apparatus defines a basin which is shaped to receive a container (B). The items to be sterilized are placed within container (B) which in turn is placed in the basin. The lid is then closed and the items are sterilized within container (B). When the lid is opened, the container (B) protects the newly sterilized apparatus from contamination by airborn microbes.

In accordance with a preferred form of the first aspect of the present invention, a liquid sterilizing or disinfecting apparatus (referred to herein also as "system") is provided. A sloping container-receiving basin contains a vent aperture adjacent an uppermost end, a drain aperture adjacent a lowermost end, and a liquid inlet. A lid selectively seals the basin when in a closed position and opens to provide ready access to the basin. A container (B) for receiving articles to be microbially decontaminated is removably disposed in the basin. A liquid sterilant suppy means supplies a liquid antimicrobial solution and a sterile rinse supply means supplies a sterile rinse liquid. A drain is connected with the basin drain aperture for draining antimicrobial and rinse liquids from the basin. A means is provided for selectively opening and closing the drain to drain liquids from the basin and retain liquids in the basin. A pump selectively pumps volumes of liquid antimicrobial solution from the liquid antimicrobial solution supply means and volumes of sterile rinse liquid from the rinse supply means to the liquid inlet for filling the container and displacing the air therefrom.

In a preferred form of the second aspect of the invention, the container includes an outer lower shell having a bottom surface and a peripheral wall. The shell defines a drain aperture adjacent to the basin lowermost portion and defines a liquid receiving aperture in fluid communication with the basin liquid inlet. A nozzle plate defines a tortuous inlet path from the liquid receiving aperture to a plurality of nozzles distributed around the lower shell. The inlet path is sufficiently tortuous that migration of contaminating microbes in ambient air after removal from the basin is inhibited. A container cover has a top surface and downward depending flanges which engage the shell to close the container. At least one of the shell and cover is configured to define a tortuous vent path from at least an uppermost portion of the container when disposed in the basin such that received liquids displace substantially all air from the container through the vent path.

Advantages shown by the present invention are as follows:
(a) it facilitates effective sterilization of medical and other items with liquid sterilants;
(b) it maintains the sterile condition of the items during temporary orlonger term storage;
(c) it facilitates maintaining an organized inventory of sterilized items.

The invention may take form in various parts and arrangements of parts. There are now described, by way of example and with reference to the accompanying drawings, preferred embodiments of apparatus and container of the invention,

In the drawings:
FIGURE 1 is a perspective view of a microbial decontamination system in accordance with the present invention;
FIGURE 2 is a diagrammatic illustration of the fluid flow paths for the system of FIGURE 1;
FIGURE 3 is a top view of the container, particularly the container cover;
FIGURE 4 is a side view of the cover;
FIGURE 5 is a top view of the container lower shell with the cover removed; and
FIGURE 6 is a side view of the shell of FIGURE 5.

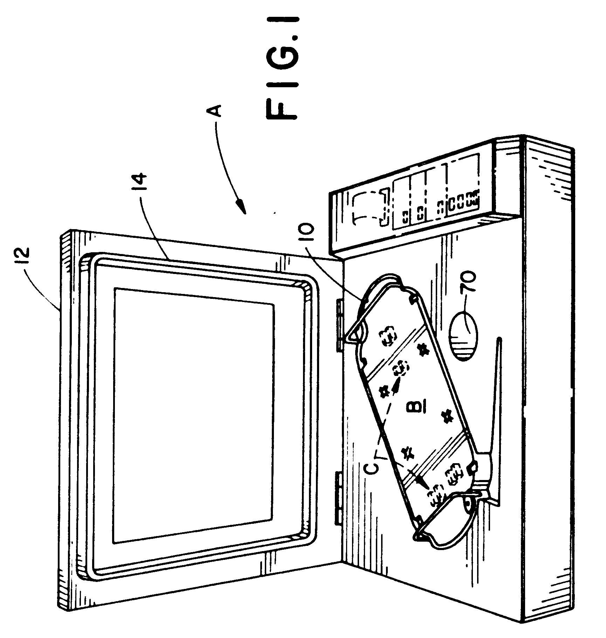

With reference to FIGURE 1, a sterilizing apparatus **A** defines a basin **10** for removably receiving one of a plurality of containers **B**. Each container may be configured or fitted with appropriate mounting structures C for holding or arranging medical instruments or other items. After the container is placed in the basin, a lid **12** is closed causing a gasket **14** to seal the basin and container from the ambient atmosphere. The sterilizing apparatus in a carefully controlled cycle floods the basin filling the container and surrounding the medical instruments, container, or other items to be sterilized with a liquid sterilant or other antimicrobial solution. The sterilant solution is drained and a sterile rinse fills the basin and container to remove chemical residue. After the rinse has been drained, the lid is opened and the container is removed as a unit containing sterilized items. The container is configured such that it permits the ingress and egress of liquid sterilant and rinse solutions but prevents microbial contamination in the ambient air from reaching the enclosed medical instruments or other items. The container with the contained sterilized items may be inventoried and stored until the sterile items are needed.

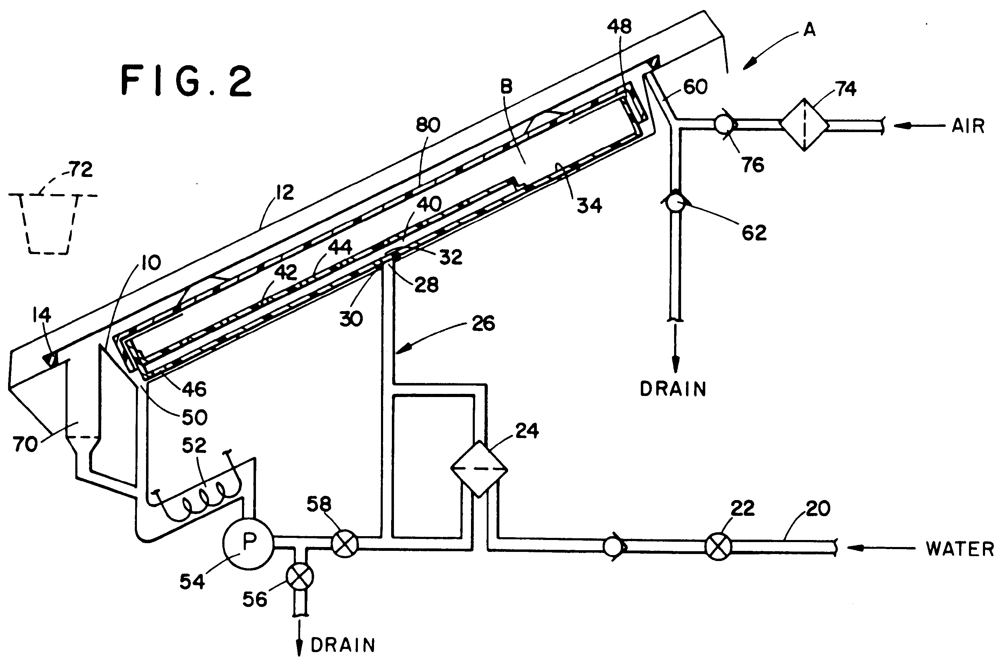

With reference to FIGURE 2, water, such as tap water, is received at an inlet **20**. A valve **22** selectively enables the water to flow to a sterilizing means **24** for sterilizing the received water. In the preferred embodiment, the sterilizing means is a filter which mechanically separates microbes and other contaminants from the received water. When the valve **22** is opened, incoming water flows through the filter **24** becoming sterilized and flows through a tubing system **26** to a basin inlet **28**. A seal **30** directs water into a liquid receiving inlet **32** in a lower wall **34** of the container **B**.

The liquid flows through a tortuous path **40** defined between the bottom wall **34** and apertures **42** in a baffle plate **44** of the container. The liquid flows into the interior of the container immersing the items to be sterilized. Liquid flows out of the container through a container drain outlet **46** and vent passage **48** and the tubing system **26** conveys the fluid from a basin drain outlet **50** to a heater **52** and a pump **54**. A drain valve **56** selectively enables the fluid to be drained from the system. A recirculating valve **58** allows the pump to pump the fluid through the tubing system back to the fluid inlet **28**. As additional sterile water flows into the system, air is displaced through a vent passage or vent outlet 60 which is connected by a check valve **62** with the drain.

The received liquid also fills a well **70** which defines a reagent receiving chamber for receiving antimicrobial agents, wetting agents, detergents, and other treatment chemicals for improving the cleaning and sterilizing effect. An ampule **72** contains peracetic acid or other strong oxidant compositions or reagents which react to form strong oxidants, as well as buffers and corrosion inhibitors to be introduced into the well. After the system is filled with water, inlet valve **22** is closed. The recirculating valve **58** is opened and the pump **54** is actuated. As fluid is drawn through the well by the pump, the reagents mix with the water forming a sterilizing or other antimicrobial solution. The sterilizing solution is circulated through the tubing system **26** back to the inlet **28** through the tortuous path **40** and distributed among the many inlets of the baffle plate **44**. As the pump **54** circulates the sterilant solution, some of the sterilant flows through the sterilizing filter **24** sterilizing it. Other solution flows through the vent **60** and other tubing and fluid contacting surfaces.

After the sterilant solution has been maintained in contact with the items in the container and all tubing valve and other surfaces between the sterilizing means **24** for a selected duration, the recirculation valve **58** is closed and the drain valve **56** is opened. The anti-microbial solution is pumped or drained from the system. In this manner, all the tubing and valve surfaces with which the sterile rinse fluid comes in contact are sterilized by the sterilant solution. More specifically, the fill valve **20** is again opened so that tap water flows through filter **24** through the sterilized tubing system **26** to the fluid inlet **28**. The sterile rinse solution continues to flow into the basin **10** until all the air is displaced. Pump **54** may be actuated again to recirculate the rinse solution for a preselected duration. Thereafter, the drain valve **56** is opened and the rinse solution is drained. Air flows through a check valve **76** and the sterilizing filter **74** such that sterile air fills the basin and container as the rinse and sterilant solutions are drained.

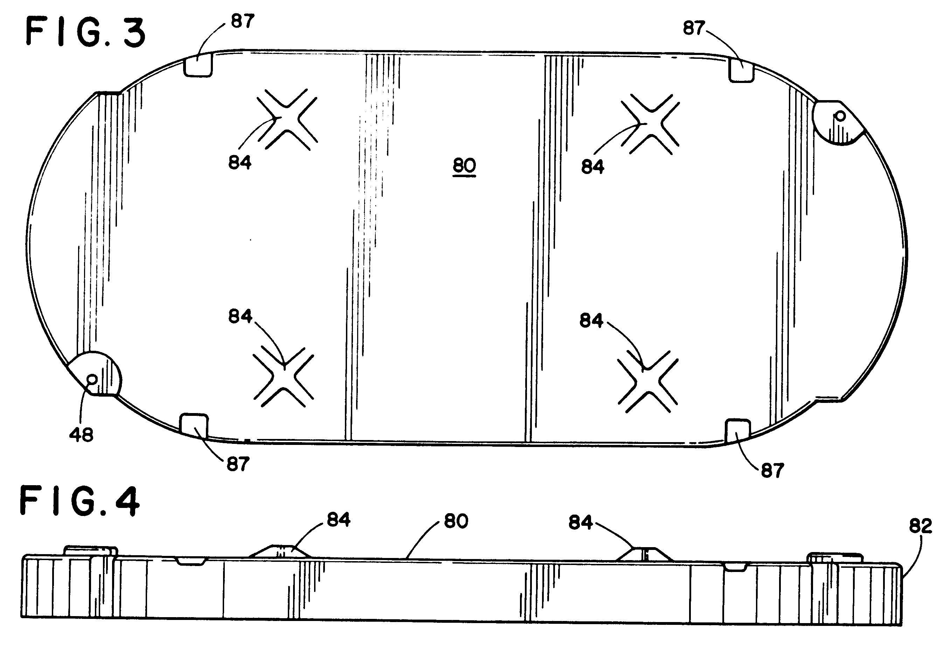

With reference to FIGURES 3 and 4, the container **B** has a generally flat cover **80** with a downward depending peripheral wall **82** extending peripherally therearound. Spacers **84** extend upward from the container cover to engage the sterilizer lid such that lid urges the container into firm engagement with the sealing means **28**. The spacers are configured such that no air is trapped by the spacers or the interaction between the spacers and the sterilizer lid. Rather, all surfaces around the spacers become immersed in the sterilant solution and are sterilized.

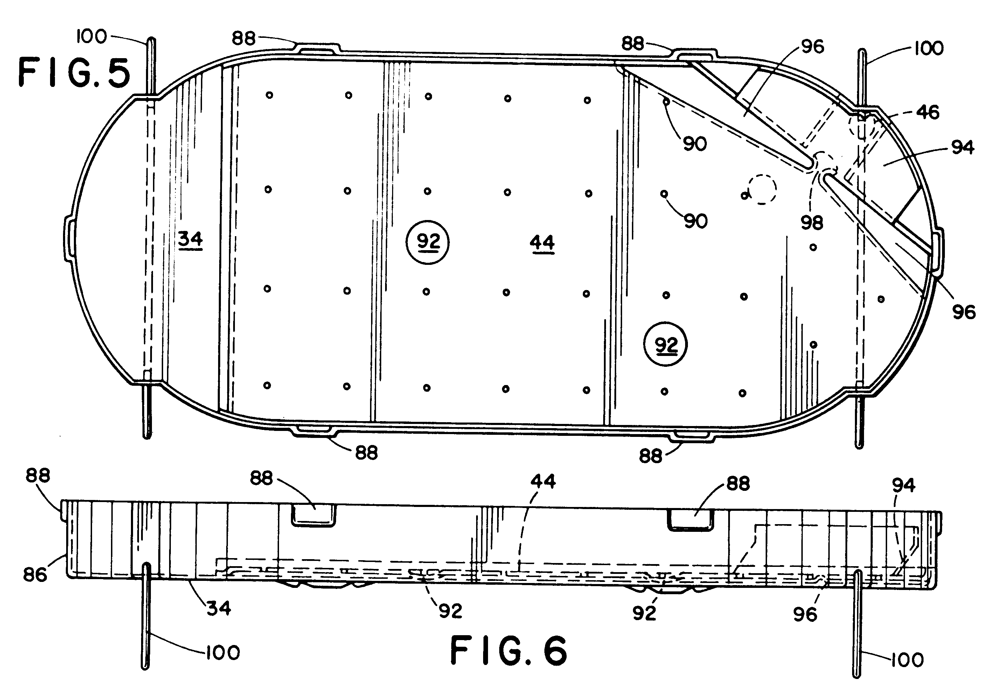

With reference to FIGURE 5, the container bottom wall **34** encloses the bottom of the container. The container bottom wall connects with a container peripheral wall **86** which extends peripherally around the container bottom wall. Projections **87** (Figure 3) define a horizontal vent path portion over the container upper lip and projections **88** define a vertical vent path portion along the side of the container so that the sterilant and rinse liquids can displace the ambient air. Moreover, the container and cover peripheral walls define the horizontal and vertical portions of the vent path **48** sufficiently tortuously that air born microbes are not carried into the container interior through the vent path.

The baffle plate **44** has a plurality of apertures **90** distributed therearound. The apertures are disposed offset from the inlet aperture **28**. In this manner, the tortuous path **40** which is defined between the inlet aperture and the baffle plate nozzle apertures is sufficiently tortuous that air borne microbes are inhibited from entering the container. The baffle plate includes a plurality of projections **92** which project downward toward the container bottom wall to maintain the baffle plate and the container in the spaced relationship. The baffle plate also defines a canted surface section **94** adjacent the drain outlet. The canted surface portion extends over the drain outlet and provides fluid flow paths towards either edge thereof. Preferably, the canted portion is symmetrically disposed over the outlet aperture **46**. Liquid then flows to the edges of the canted portion onto the lower surface **34** and down to the drain aperture **46**. In this manner, a tortuous path is again defined between the drain aperture and the interior of the container. Projections **96** provide a barrier between the inlet and outlet passages so that the liquid pumped into the inlet flows through the baffle plate nozzle apertures primarily rather than directly to the outlet. A small vent passage **98** is defined through the barrier to assure that no ambient air is trapped adjacent the vent outlet and to thoroughly drain the baffle interspace during draining cycles. When the pump **54** is activated, some liquid will, of course, flow through this barrier vent but the majority will be discharged through the baffle plate apertures **90**.

A pair of handles **100** are interconnected with the container lower portion to facilitate lifting of the container without accidental removal of the container cover.

The invention has been described with reference to the preferred embodiment. Obviously, modifications and alterations will occur to others upon reading and understanding the preceding detailed description. For example, the bottom wall and the baffle plate may be designed with appropriate ridges, partitions, depressions, and the like such that the container is dedicated to accommodating a preselected type of medical apparatus, items, or other equipment. Larger and smaller containers may also be provided. A plurality of smaller containers might even be provided within the basin and have their contents sterilized concurrently. It is intended that the invention be construed as including all such alterations and modifications insofar as they come within the scope of the appended claims or the equivalents thereof.

## Claims

1. Apparatus for use in the microbial decontamination of an article using an antimicrobial liquid, characterised in that the apparatus comprises:
a basin (10) having
(i) a vent aperture (60) disposed in an upper portion thereof,
(ii) a drain aperture (50) disposed in a lower portion thereof, and
(iii) an inlet (28) for the antimicrobial liquid;
a lid (12) for selectively sealing the top of the basin when in a closed position and providing ready access to the basin in an open position;
a container (B) for receiving said article to be microbially decontaminated, the container being removably disposed in the basin, the container defining a container liquid receiving inlet (32), a container drain aperture (46), and a nozzle plate (44) which defines a tortuous inlet path (40) between the container liquid inlet and a plurality of apertures (90) defined in and distributed around the nozzle plate, the tortuous inlet path being sufficiently tortuous that a migration of contaminating microbes in ambient air after removal from the basin is inhibited;
an antimicrobial liquid supply means (20, 70, 72) for supplying the antimicrobial liquid;
a rinse liquid supply means (20) for supplying a rinse liquid;
a drain (46) operatively connected with the drain aperture (50) of the basin for draining the antimicrobial liquid and the rinse liquid from the basin;
a means (56) for opening and closing the drain selectively to drain liquid from the basin and to retain liquid in the basin; and
a pump (54) for selectively pumping volumes of antimicrobial liquid from the antimicrobial liquid supply means and rinse liquid from the rinse liquid supply means to the inlet (28) for selectively filling the container (B) and displacing ambient air therefrom.

2. A container for use in the apparatus of Claim 1, characterised in that said container comprises:
a lower shell and a cover therefor;
the lower shell having a bottom (34) and a peripheral wall (86), there being disposed in the bottom (34) a liquid-receiving inlet (32) and a drain aperture (46) for the antimicrobial liquid, the drain aperture being disposed in a first region of the bottom (34) and adjacent the peripheral wall (86);
a plate (44) which, together with bottom (34) defines a tortuous inlet path (40) between the liquid-receiving inlet (32) and a plurality of apertures (90) disposed in and distributed over the plate;
the inlet path (40) being sufficiently tortuous that migration of contaminating microbes from ambient air is inhibited:
the container cover having a top (80) and a downward depending peripheral wall (82) which engages the lower shell to close the container; and
at least one of the shell and cover being configured to define a tortuous vent path (48) adjacent a second region of the lower shell spaced apart from said first region and such that liquid received in the container displaces air from the container along the vent path (48).

3. Apparatus according to Claim 1, in which the container (B) is as claimed in Claim 2.

4. Apparatus according to Claim 3, in which the plate includes a generally flat surface and said apertures (90) are disposed as an array in the plate with respect to the inlet (32) and drain aperture (46) so that there is no straight flow path connecting the inlet, the drain aperture and any one of the apertures (90).

5. Apparatus according to Claim 3 or 4, in which plate (44) includes a canted portion (94) extending over the drain aperture (46), the plate being mounted in a spaced relationship from the bottom (34) such that liquid flowing through the container inlet (32) can flow through a narrow passage between the bottom and the plate to the apertures (90), the canted portion defining at least one passage into the narrow space beteween the plate and the shell bottom to provide access to the drain aperture.

6. Apparatus according to Claim 3, 4 or 5, which includes a partition (96) extending between the plate and shell bottom between the inlet and drain apertures to inhibit fluid from flowing directly from the inlet aperture to the drain aperture.

7. Apparatus according to Claim 6, in which the partition (96) includes a small vent aperture (98) therethrough to prevent air from becoming trapped adjacent the drain aperture between the shell bottom and the plate.

8. Apparatus according to any of Claims 3 to 7, in which the cover peripheral wall (82) circumscribes the shell peripheral wall (86) in a spaced relationship to define the vent path (48).

9. Apparatus according to Claim 8, which includes spacers mounted to one of the shell and cover peripheral walls to hold the shell and cover peripheral walls in said spaced relationship.

10. Apparatus according to any of Claims 3 to 9, in which the cover is transparent such that articles held in the container can be viewed from the exterior without removing the cover.

11. Apparatus according to any of Claims 3 to 10, which includes instrument holders (C) disposed on the plate for supporting and retaining the articles.

12. Apparatus according to any of Claims 3 to 11, which includes a handle means for assisting an operator to engage and lift the lower shell without disrupting the cover.

13. Apparatus for use in the microbial decontamination of an article using an antimicrobial liquid, characterised in that the apparatus comprises:
a container-receiving basin (10), the basin having a drain aperture (50) adjacent a lower portion thereof and a liquid inlet (28);
a lid (12) for selectively sealing the basin in a closed position and for providing access to the basin in an open position;
a liquid antimicrobial supply means for supplying an antimicrobial liquid;
a rinse supply means for supplying a rinse liquid;
a drain operatively connected with the drain aperture (50) for draining antimicrobial and rinse liquids from the basin;
a tubing system (26) interconnecting the antimicrobial supply means, the rinse supply means and the liquid inlet (28);
a valve (56) for selectively opening and closing the drain to drain liquid from the basin and retain liquid in the basin;
a pumping means (54) operatively connected with the tubing system (26) for selectively pumping at least the antimicrobial liquid;
a container apparatus (B) removably disposed in the basin for holding an item to be microbially decontaminated with the antimicrobial liquid, said container comprising a lower shell and a cover therefor;
the lower shell having a bottom surface (34), a shell peripheral wall (86) and a drain aperture (50) adjacent the basin lowermost portion;
the drain aperture (50) being adjacent a junction between the bottom surface (34) and the shell peripheral wall (86) adjacent a first end of the shell;
the lower shell defines a liquid-receiving inlet (32) in fluid communication with the basin liquid inlet (28);
a nozzle plate (44) which defines a tortuous inlet path (40) between the shell liquid-receiving inlet (32) and a plurality of nozzles or other apertures (90) defined in and distributed around the plate;
the inlet path (40) being sufficiently tortuous that a migration of contaminating microbes from ambient air after removal from the basin is inhibited;
the container cover (80) having a top surface and a downward depending cover peripheral wall (82) which engages the lower shell to close the container; and
at least one of the shell and cover being configured to define a tortuous vent path (48) adjacent a second end opposite the shell first end, from at least an uppermost portion of the container to the basin such that received liquids displace substantially all air from the container through the vent path (48).

## Patentansprüche

1. Vorrichtung zum Verwenden beim mikrobischen Dekontaminieren eines Gegenstandes unter Verwendung einer antimikrobischen Flüssigkeit, **dadurch gekennzeichnet,** daß die Vorrichtung aufweist:
ein Becken (10) mit
(i) einer Lüftungsöffnung (60), welche in einem oberen Bereich derselben angeordnet ist,
(ii) eine Abschlußöffnung (50), welche in einem unteren Abschnitt derselben angeordnet ist, und
(iii) einen Einlaß (28) für die antimikrobische Flüssigkeit;
einen Deckel (12) zum wahlweisen Dichten der Oberseite des Beckens in einer geschlossenen Stellung und zum Ermöglichen eines schnellen Zugriffs zu dem Becken in einer offenenen Stellung;
einen Behälter (B) für die Aufnahme des Gegenstandes, welcher mikrobisch dekontaminiert werden soll, wobei der Behälter entfernbar in dem Becken anordenbar ist, wobei der Behälter einen Behälterflüssigkeitsaufnahme-Einlaß (32), eine Behälterabflußöffnung (46), und eine Düsenplatte (44) bestimmt, welche einen gewundenen Einlaßpfad (40) zwischen dem Behälterflüssigkeitseinlaß und einer Vielzahl von Öffnungen (90) bestimmt, welche in der Düsenplatte bestimmt und um die Düsenplatte verteilt sind, wobei der gewundene Einlaßpfad ausreichend gewunden ist, so daß ein Wandern der kontaminierenden Mikroben in die Umgebungsluft nach Entfernen aus dem Becken gehemmt ist;
eine Antimikrobenflüssigkeitsversorgungseinrichtung (20, 70, 72) für das Zuführen der antimikrobischen Flüssigkeit;
eine Spülflüssigkeitsversorgungseinrichtung (20) für das Bereitstellen einer Spülflüssigkeit;
einen Abfluß (46), welcher wirksam mit der Abflußöffnung (50) des Beckens für das Abfließen der antimikrobischen Flüssigkeit und der Spülflüssigkeit aus dem Becken verbunden ist;
Mittel (56) zum wahlweisen Öffnen und Schließen des Abflusses zum Abfließen von Flüssigkeit aus dem Becken und zum Zurückhalten von Flüssigkeit in dem Becken; und
eine Pumpe (54) zum wahlweisen Pumpen von Volumina von antimikrobischer Flüssigkeit von der Antimikrobenflüssigkeitsversorgungszufuhreinrichtung und der Spülflüssigkeit von der Spülflüssigkeitsversorgungseinrichtung zu dem Einlaß (28) zum wahlweisen Füllen des Behälters (B) und zum Verdrängen von Umgebungsluft daraus.

2. Ein Behälter zum Verwenden in einer Vorrichtung nach Anspruch 1, **dadurch gekennzeichnet,** daß der Behälter aufweist:
eine untere Schale und eine Abdeckung hierfür;
wobei die untere Schale einen Boden (34) und eine Umfangswand (86) hat, wobei in dem Boden (34) ein Flüssigkeitsaufnahme-Einlaß (32) und eine Abflußöffnung (46) für die antimikrobische Flüssigkeit angeordnet sind, wobei die Abflußöffnung in einem ersten Bereich des Bodens (34) und angrenzend an die Umfangswand (86) angeordnet ist;
eine Platte (44), welche, zusammen mit dem Boden (34) einen gewundenen Einlaßpfad 40) zwischen dem Flüssigkeitsaufnahme-Einlaß (32) und einer Vielzahl von Öffnungen (90) bestimmt, welche in der und verteilt über die Platte angeordnet sind;
wobei der Einlaßpfad (40) so ausreichend gewunden ist, daß ein Wandern von kontaminierenden Mikroben aus der Umgebungsluft gehemmt ist;
wobei die Behälterabdeckung, ein Oberteil (80) und eine nach unten weisende Umfangswand (82) hat, welche an der unteren Schale angreift um den Behälter zu schließen; und
wobei wenigstens die Schale oder die Abdeckung so gestaltet ist, daß ein gewundener Entlüftungspfad (48), benachbart einem zweiten Bereich der unteren Schale im Abstand von dem ersten Bereich bestimmt und so angeordnet ist, daß Flüssigkeit, welche in dem Behälter aufgenommen ist, Luft aus dem Behälter entlang des Lüftungspfades (48) verdrängt.

3. Vorrichtung nach Anspruch 1, bei welcher der Behälter (B) so ist wie in Anspruch 2 beansprucht.

4. Vorrichtung nach Anspruch 3, bei welcher die Platte eine im wesentlichen flache Oberfläche einschließt und die Öffnung (90) in einer Gruppe so in Beziehung zu dem Einlaß (32) und der Abflußöffnung (46) angeordnet sind, daß dort kein gerader Strömungpfad vorhanden ist, welcher den Einlaß, die Abflußöffnung und irgendeine der Öffnungen (90) verbindet.

5. Vorrichtung nach Anspruch 3 oder 4, in welcher die Platte (44) einen abgekanteten Abschnitt (94) einschließt, welcher sich über die Abflußöffnung (46) erstreckt, wobei die Platte in Abstandsbeziehung zu dem Boden (34) eingebaut ist, so daß ein Flüssigkeit, welche durch den Behältereinlaß (32) strömt, durch einen engen Durchgang zwischen dem Boden und der Platte zu den Öffnungen (90) fließen kann, wobei der abgekantete Bereich wenigstens einen Durchgang in den engen Raum zwischen der Platte und dem Schalenboden aufweist, um einen Zugang zu der Abflußöffnung vorzusehen.

6. Vorrichtung nach den Ansprüchen 3, 4 oder 5, welche einen Abteilung (96) aufweist, welche sich zwischen der Platte und dem Schalenboden zwischen dem Einlaß und der Abflußöffnung erstreckt, um Fluid zu hindern, direkt von der Einlaßöffnung zu der Abflußöffnung zu strömen.

7. Vorrichtung nach Anspruch 6, bei welcher die Abteilung (96) eine kleine Lüftungsöffnung (98) aufweist, wodurch Luft gehindert wird, benachbart der Abflußöffnung zwischen dem Schalenboden und der Platte eingefangen zu werden.

8. Vorrichtung nach einem der Ansprüche 3 bis 7, bei welcher die Umfangswand (82) der Abdeckung die Umfangswand (86) der Schale in einem räumlichen Abstand umgibt, um einen Entlüftungspfad (48) zu bestimmen.

9. Vorrichtung nach Anspruch 8, welche Abstandhalter aufweist, welche an einer der Unfangswände der Schale und Abdeckung angeordnet sind, um die Unfangswände der Schale und der Abdeckung in dem räumlichen Abstand zu halten.

10. Vorrichtung nach einem der Ansprüche 3 bis 9, bei welcher die Abdeckung derart transparent ist, daß Gegenstände, welche in dem Behälter gehalten werden, von Außen ohne Entfernen der Abdeckung betrachtet werden können.

11. Vorrichtung nach einem der Ansprüche 3 bis 10, welche Instrumentenhalter (C) aufweist, welche auf der Platte zum Abstützung und zum Zurückhalten der Gegenstände angeordnet sind.

12. Vorrichtung nach einem der Ansprüche 3 bis 11, welche Handhabungsmittel zum Unterstützen einer Bedienungsperson beim Ergreifen und Anheben der unteren Schale ohne Zerstören der Abdeckung aufweist.

13. Vorrichtung zum Verwenden bei dem mikrobischen Dekontaminieren eines Gegenstandes unter Verwendung einer anitmikrobischen Flüssigkeit, **dadurch gekennzeichnet**, daß die Vorrichtung folgendes enthält:
ein Behälter-aufnehmendes Becken (10), wobei das Becken eine seinem unteren Abschnitt benachbarte Abflußöffnung (50) und einen Flüssigkeitseinlaß (28) hat;
einen Deckel (12) zum wahlweisen Dichten des Beckens in einer geschlossenen Stellung und zum Ermöglichen eines Zuganges zu dem Becken in einer offenen Stellung;
eine Antimikrobenflüssigkeitsversorgungseinrichtung zum Zuführen einer antimikrobischen Flüssigkeit;
eine Spülversorgungseinrichtung zum Zuführen einer Spülflüssigkeit;
einen Abfluß, welcher wirksam mit der Abflußöffnung (50) zum Abfließen von antimikrobischer und Spülflüssigkeiten aus dem Becken verbunden ist;
ein Rohrsystem (26), welches die Antimikrobenflüssigkeitsversorgungseinrichtung, die Spülversorgungseinrichtung und den Flüssigkeitseinlaß (28) miteinander verbindet;
ein Ventil (56) zum wahlweisen Öffnen und Schließen des Abflusses zum Abfließen von Flüssigkeit aus dem Becken und zum Zurückhalten von Flüssigkeit in dem Becken;
eine Pumpeneinrichtung (54), welche wirksam mit dem Rohrsystem (26) zum wahlweisen Pumpen wenigstens einer antimikrobischen Flüssigkeit verbunden ist;
eine Behältervorrichtung (B), welche entfernbar in dem Becken zum Halten eines Gegenstandes, welcher mittels der antimikrobischen Flüssigkeit mikrobisch dekontaminiert werden soll, angeordnet ist, wobei der Behälter eine untere Schale und eine Abdeckung für diese aufweist;
wobei die untere Schale eine Bodenoberfläche (34), eine Schalenumfangswand (86) und eine Abflußöffnung (50), welche an den untersten Abschnitt des Beckens angrenzt, hat;
wobei die Abflußöffnung (50) benachbart einer Verbindung zwischen der Bodenoberfläche (34) und der Schalenumfangswand (86)benachbart einem ersten Ende der Schale ist;
wobei die untere Schale einen Flüssigkeitsaufnahme-Einlaß (32), welcher in Fluidverbindung mit dem Beckenflüssigkeitseinlaß steht, bestimmt eine Düsenplatte (44), welche einen gewundenen Einlaßpfad (40) zwischen dem Schalenflüssigkeitsaufnahme-Einlaß (32) und einer Vielzahl von Düsen oder anderen Öffnungen (90) bestimmt, welche in der Platte bestimmt und um die Platte verteilt sind;
wobei der Einlaßpfad (40) so ausreichend gewunden ist, daß ein Wandern von kontaminierenden Mikroben aus der Umgebungsluft nach Entfernen aus dem Becken verhindert wird;
wobei die Behälterabdeckung (80) eine Oberteilfläche und eine sich nach unten erstreckende Abdeckungsumfangswand (82) hat, welche an der unteren Schale zum Schließen des Behälters angreift; und
wobei wenigstens die Schale oder die Abdeckung so gestaltet ist, daß eine gewundener Lüftungspfad (48) benachbart einem zweiten Ende gegenüber dem ersten Ende der Schale bestimmt ist, von wenigstens einem obersten Abschnitt des Behälters zu dem Becken, so daß aufgenommene Flüssigkeiten im wesentlichen die gesamte Luft durch den Lüftungspfad (48) aus dem Behälter verdrängt.

## Revendications

1. Appareil susceptible d'être utilisé dans la décontamination microbienne d'un article en utilisant un liquide antimicrobien, caractérisé en ce que l'appareil comprend :
une cuvette (10) comportant :
(i) une ouverture d'évacuation (60) disposée dans sa partie supérieure,
(ii) une ouverture de décharge (50) disposée dans sa partie inférieure, et
(iii) une entrée (28) pour le liquide antimicrobien,
un couvercle (12) pour sceller de manière sélective la partie supérieure de la cuvette en position de fermeture et assurer un accès aisé à la cuvette en position d'ouverture,
un récipient (B) pour recevoir ledit article à décontaminer contre les microbes, le récipient étant disposé de manière amovible dans la cuvette, le récipient définissant une entrée réceptrice de liquide (32), une ouverture de décharge (46) et une plaque à buses (44) qui définit un chemin d'entrée tortueux (40) entre l'entrée de liquide du récipient et une série d'ouvertures (90) définies et distribuées autour de la plaque à buses, le chemin d'entrée tortueux étant suffisamment tortueux pour inhiber une migration des microbes contaminants dans l'air ambiant après retrait de la cuvette,
des moyens d'alimentation en liquide antimicrobien (20, 70, 72) pour acheminer le liquide antimicrobien,
des moyens d'alimentation en liquide de rinçage (20) pour acheminer un liquide de rinçage,
un drain (46) coopérant avec l'ouverture de décharge (50) de la cuvette pour décharger le liquide antimicrobien et le liquide de rinçage de la cuvette,
des moyens (56) pour ouvrir et fermer le drain sélectivement pour évacuer le liquide de la cuvette et retenir le liquide dans la cuvette, et
une pompe (54) pour pomper de manière sélective des volumes de liquide antimicrobien des moyens d'alimentation en liquide antimicrobien et de liquide de rinçage de moyens d'alimentation en liquide de rinçage vers l'entrée (28) pour remplir de manière sélective le récipient (B) et en déplacer l'air ambiant.

2. Récipient susceptible d'être utilisé dans l'appareil de la revendication 1, caractérisé en ce que ledit récipient comprend :
une enveloppe inférieure et un couvercle qui lui est destiné,
l'enveloppe inférieure ayant un fond (34) et une paroi périphérique (86), le fond (34) recevant une entrée de réception de liquide (32) et une ouverture de décharge (46) pour le liquide antimicrobien, l'ouverture de décharge étant disposée dans une première région du fond (34) au voisinage de la paroi périphérique (86),
une plaque (44) qui, conjointement avec le fond, définit un chemin d'entrée tortueux (40) entre l'entrée de réception de liquide (32) et une série d'ouvertures (90) disposées et distribuées sur la plaque,
le chemin d'entrée (40) étant suffisamment tortueux pour inhiber la migration de microbes contaminants de l'air ambiant,
le couvercle du récipient ayant une partie supérieure (80) et une paroi péripérique (82) qui pend vers le bas et qui vient en contact avec l'enveloppe inférieure pour fermer le récipient, et
au moins l'enveloppe ou le couvercle ayant une configuration permettant de définir un chemin d'échappement tortueux (48) au voisinage d'une deuxième région de l'enveloppe inférieure espacée de ladite première région et telle que le liquide reçu dans le récipient déplace l'air de celui-ci le long du chemin d'échappement (48).

3. Appareil selon la revendication 1, dans lequel le récipient (B) est tel que décrit dans la revendication 2.

4. Appareil selon la revendication 3, dans lequel la plaque comprend une surface de forme générale plate et lesdites ouvertures (90) sont disposées en réseau dans la plaque par rapport à l'entrée (32) et à l'ouverture de décharge (46) de telle sorte qu'il n'y ait pas de chemin d'écoulement rectiligne connectant l'entrée, l'ouverture de décharge et l'une quelconque des ouvertures (90).

5. Appareil selon la revendication 3 ou 4, dans lequel la plaque (44) comprend une partie chanfreinée (94) s'étendant au-dessus de l'ouverture de décharge (46), la plaque étant montée à distance du fond (34) de telle sorte que le liquide s'écoulant par l'entrée (32) du récipient puisse s'écouler à travers un passage étroit entre le fond et la plaque vers les ouvertures (90), la partie chanfreinée définissant au moins un passage dans l'espace étroit entre la plaque et le fond de l'enveloppe pour assurer un accès à l'ouverture de décharge.

6. Appareil selon la revendication 3, 4 ou 5, qui comprend une cloison (96) s'étendant entre la plaque et le fond de l'enveloppe, entre les ouvertures d'entrée et de décharge, pour empêcher le fluide de s'écouler directement de l'ouverture d'entrée à l'ouverture de décharge.

7. Appareil selon la revendication 6, dans lequel la cloison (96) comprend une petite ouverture d'échappement (98) qui la traverse pour empêcher l'air d'être piégé au voisinage de l'ouverture de décharge entre le fond de l'enveloppe et la plaque.

8. Appareil selon l'une quelconque des revendications 3 à 7, dans lequel la paroi périphérique (82) du couvercle circonscrit la paroi périphérique (86) de l'enveloppe selon un certain espacement pour définir le chemin d'échappement (48).

9. Appareil selon la revendication 8, qui comprend des éléments d'espacement montés sur l'une des parois périphériques de l'enveloppe et du couvercle pour maintenir les parois périphériques de l'enveloppe et du couvercle dans ledit espacement.

10. Appareil selon l'une quelconque des revendications 3 à 9, dans lequel le couvercle est transparent si bien que les articles maintenus à l'intérieur du récipient peuvent être observés de l'extérieur sans enlever le couvercle.

11. Appareil selon l'une quelconque des revendications 3 à 10, qui comprend des porte-instruments (C) disposés sur la plaque pour supporter et maintenir les articles.

12. Appareil selon l'une quelconque des revendications 3 à 11, qui comprend des moyens à poignée pour aider un opérateur à tenir et à soulever l'enveloppe inférieure sans déranger le couvercle.

13. Appareil susceptible d'être utilisé pour la décontamination microbienne d'un article en utilisant un liquide antimicrobien, caractérisé en ce que l'appareil comprend :
une cuvette (10) recevant un récipient, la cuvette ayant une ouverture de décharge (50) au voisinage de sa partie inférieure et une entrée de liquide (28),
un couvercle (12) pour sceller de manière sélective la cuvette en position de fermeture et pour fournir un accès à la cuvette en position d'ouverture,
des moyens d'alimentation en liquide antimicrobien pour alimenter en liquide antimicrobien,
des moyens d'alimentation en liquide de rinçage pour alimenter en liquide de rinçage,
un drain coopérant avec l'ouverture de décharge (50) pour évacuer le liquide antimicrobien et le liquide de rinçage de la cuvette,
un système de canalisations (26) interconnectant les moyens d'alimentation en liquide antimicrobien, les moyens d'alimentation en liquide de rinçage et l'entrée de liquide (28),
une vanne (56) pour ouvrir et fermer sélectivement le drain afin de décharger le liquide de la cuvette et de retenir le liquide dans la cuvette,
un moyen de pompage (54) coopérant avec le système de canalisations (26) pour pomper de manière sélective au moins le liquide antimicrobien,
un appareil à récipient (B) disposé de manière amovible dans la cuvette pour maintenir un article qui doit être soumis à une décontamination microbienne par le liquide antimicrobien, ledit récipient comprenant une enveloppe inférieure et un couvercle destiné à celle-ci,
l'enveloppe inférieure ayant une surface inférieure (34), une paroi périphérique (86) et une ouverture de décharge (50) au voisinage de la partie la plus basse de la cuvette,
l'ouverture de décharge (50) étant voisine d'une jonction entre la surface inférieure (34) et la paroi périphérique de l'enveloppe (86) à proximité d'une première extrémité de l'enveloppe,
l'enveloppe inférieure définit une entrée de réception de liquide (32) en communication de fluide avec l'entrée de liquide de la cuvette (28),
une plaque à buses (44) qui définit un chemin d'entrée tortueux (40) entre l'entrée de réception de liquide (32) de l'enveloppe et une série de buses ou d'autres ouvertures (90) définies et distribuées autour de la plaque,
le chemin d'entrée (40) étant suffisamment tortueux pour inhiber une migration de microbes contaminants de l'air ambiant après retrait de la cuvette,
le couvercle (80) du récipient ayant une surface supérieure et une paroi périphérique (82) pendant vers le bas qui vient en contact avec l'enveloppe inférieure pour fermer le récipient, et
au moins l'enveloppe ou le couvercle étant configuré(e) pour définir un chemin d'échappement tortueux (48) au voisinage d'une deuxième extrémité opposée à la première extrémité de l'enveloppe, d'au moins une partie supérieure du récipient à la cuvette de telle sorte que le liquide reçu déplace sensiblement tout l'air provenant du récipient à travers le chemin d'échappement (48).
